# EUROPEAN PATENT SPECIFICATION

(11) **EP 4 486 256 B1**
(45) Date of publication and mention of the grant of the patent: **01.07.2026**
(21) Application number: 23712727.9
(22) Date of filing: 23.02.2023
(51) Int. Cl.: A61F 2/26, A61F 2/00

(54) **AN IMPLANTABLE DEVICE FOR SIZING AN INFLATABLE MEMBER**
IMPLANTIERBARE VORRICHTUNG ZUR GRÖSSENBESTIMMUNG EINES AUFBLASBAREN ELEMENTS
DISPOSITIF IMPLANTABLE POUR DIMENSIONNEMENT D'UN ÉLÉMENT GONFLABLE

(30) Priority: 28.02.2022 US 202263268647 P; 22.02.2023 US 202318172939
(43) Date of publication of application: 08.01.2025
(73) Proprietor: Boston Scientific Scimed Inc., Maple Grove, Minnesota 55311-1566 (US)
(72) Inventor: WATSCHKE, Brian P., Minneapolis, Minnesota 55405 (US)
(74) Representative: Pfenning, Meinig & Partner mbB
(86) International application number: PCT/US2023/063110
(87) International publication number: WO 2023/164535

(56) References cited:
- US-A1- 2020 222 188
- US-B2- 10 874 516

## Description

### TECHNICAL FIELD

This disclosure relates generally to an implantable device having an electronic pump assembly that can determine sizing information for an inflatable member.

### BACKGROUND

An artificial urinary sphincter device or an inflatable penile prosthesis includes an inflatable member (e.g., a cuff or cylinder(s)). The inflatable member may have a plurality of different sizes that can be implanted in the patient. With respect to the urinary sphincter device, a tape or cuff sizing tool may be utilized to measure the circumference of the urethra and indicate what cuff size to be used in the procedure. In some examples, the tape is flexible (and the urethral tissue is compressible), which may cause sizing and cuff selection relatively difficult. With respect to the inflatable penile prosthesis, a physician may select a size of the cylinder(s) based on a visual inspection and the ability to dilate the tunica, where physicians may perform the sizing selection in different ways. Incorrect selection of the size of the inflatable member may lead to poor performance or injury to the patient.

A device according to the preamble of claim 1 is known from the document US 2020/222188 A.

### SUMMARY

The present invention relates to an implantable device comprising a fluid reservoir configured to hold fluid and an inflatable member configured to be implanted in a body of a patient as set forth in the appended claims.

According to an aspect, an implantable device includes a fluid reservoir configured to hold fluid, an inflatable member configured to be implanted in a body of a patient, and an electronic pump assembly. The electronic pump assembly includes a controller configured to actuate at least one of a pump or an active valve to inflate the inflatable member, and a pressure sensor configured to monitor a pressure of the inflatable member. The controller is configured to detect a value of at least one fluid transfer parameter for the pressure of the inflatable member to reach a target pressure during an inflation cycle. The controller is configured to generate size capability information of the inflatable member for the patient based on the detected value of the at least one fluid transfer parameter.

According to the present invention, the size capability information indicates whether a size of the inflatable member is compatible with the patient. The size capability information indicates a sizing option for the inflatable member for the patient. The controller is configured to transmit the size capability information to an external device. The controller includes a memory device storing sizing information, where the sizing information defines at least one sizing option, and the at least one sizing option is mapped to a range of values of the at least one fluid transfer parameter. The controller is configured to determine the size capability information based on whether the detected value is included within the range of values of the at least one fluid transfer parameter. The at least one fluid transfer parameter includes a time duration to achieve the target pressure. The at least one fluid transfer parameter includes a volume of the fluid transferred to the inflatable member to achieve the target pressure. The at least one fluid transfer parameter includes a pump rate required to achieve the target pressure in a predetermined amount of time.

According to the present invention, the implantable device includes a fluid reservoir configured to hold fluid, an inflatable member, and an electronic pump assembly. The electronic pump assembly includes a controller configured to actuate at least one of a pump or an active valve to inflate the inflatable member. The controller includes a memory device storing sizing information. The sizing information defines one or more of a plurality of sizing options. Each sizing option is mapped to a range of values of at least one fluid transfer parameter. The electronic pump assembly includes a pressure sensor configured to monitor a pressure of the inflatable member. The controller is configured to detect a value of the at least one fluid transfer parameter for the pressure of the inflatable member to reach a target pressure during an inflation cycle. The controller is configured to select a sizing option from the plurality of sizing options based on the detected value of the at least one fluid transfer parameter.

According to some aspects, the implantable device may include one or more features (or any combinations thereof). The plurality of sizing options include a first sizing option and a second sizing option. The first sizing option is mapped to a first range of values. The second sizing option is mapped to a second range of values. The controller is configured to select the first sizing option in response to the detected value belonging to the first range of values. The at least one fluid transfer parameter includes a time duration to achieve the target pressure, a volume of the fluid transferred to the inflatable member to achieve the target pressure, or a pump rate required to achieve the target pressure in a predetermined amount of time. The controller is configured to detect a performance issue event based on the value of the at least one fluid transfer parameter for the pressure of the inflatable member to reach the target pressure during the inflation cycle. The controller is configured to detect a tissue atrophy event based on the value of the at least one fluid transfer parameter for the pressure of the inflatable member to reach the target pressure during the inflation cycle. The implantable device includes a urinary sphincter device. The implantable device includes an inflatable penile prosthesis. The controller is configured to transmit the selected sizing option to an external device for display.

Also disclosed but not part of the present invention, is a method for determining size capability information of an implantable device includes actuating at least one of a pump or an active valve of an electronic pump assembly to transfer fluid from a fluid reservoir to an inflatable member during an inflation cycle, where the inflatable member is implanted in a body of a patient, monitoring, by a pressure sensor of the electronic pump assembly, a pressure of the inflatable member in the body of the patient, detecting a value of at least one fluid transfer parameter for the pressure of the inflatable member to reach a target pressure during the inflation cycle, and generating size capability information of the inflatable member for the patient based on the detected value of the at least one fluid transfer parameter. In some examples, the method may include transmitting the size capability information to an external device. The size capability information indicates at least one of whether a size of the inflatable member is compatible with the patient or a sizing option for the inflatable member for the patient.

### BRIEF DESCRIPTION OF THE DRAWINGS

FIG. 1A illustrates an implantable device having an electronic pump assembly according to an aspect.
FIG. 1B illustrates a controller of the electronic pump assembly according to an aspect.
FIG. 1C illustrates an example of sizing information that is used to determine size capability information according to an aspect.
FIG. 2 illustrates a pressure profile depicting sizing information for a plurality of sizing options according to an aspect.
FIG. 3 illustrates a pressure profile depicting sizing information indicating whether an inflatable member is compatible with a patient according to an aspect.
FIG. 4 illustrates a pressure profile depicting sizing information for an inflatable member of a penile prosthesis according to an aspect.
FIG. 5 illustrates a pressure profile depicting a tissue atrophy event for an inflatable member according to an aspect.
FIG. 6 illustrates a flow chart depicting example operations of generating sizing capability information for an inflatable member of an implantable device according to an aspect.
FIG. 7 illustrates an example of a urinary control device according to an aspect.
FIG. 8 illustrates an example of an inflatable penile prosthesis according to an aspect.

### DETAILED DESCRIPTION

This disclosure relates to an implantable device that can automatically determine size capability information of an inflatable member implanted in the body of a patient. In some examples, the size capability information may indicate whether a size of the inflatable member is compatible with the patient. In some examples, the size capability information may indicate a sizing option for the inflatable member for the patient. The size capability information may be transmitted to an external device for display.

In some examples, the implantable device is an artificial urinary sphincter device. In some examples, the implantable device is an inflatable penile prosthesis. The implantable device includes an inflatable member, an electronic pump assembly, and a fluid reservoir. The electronic pump assembly may transfer fluid between the fluid reservoir and the inflatable member without the user manually operating a pump bulb.

The electronic pump assembly includes a controller, one or more active valves, one or more pumps, and one or more pressure sensors. The controller may receive pressure readings from the pressure sensor and control the pump(s) and the active(s) valves to maintain and/or adjust the pressure of the inflatable member. The controller may send control signals to the pump(s) and the active valve(s) to inflate or deflate the inflatable member. In some examples, the control of the inflation state and the deflation state is based on wireless signals received from an external device that is operated by the patient (and the detected pressure of the inflatable member from the pressure sensor(s)). For example, the patient may use the external device to place the inflatable member in an inflation or deflation state, which causes the external device to send a command to the controller on the electronic pump assembly.

The controller includes a sizing module configured to generate size capability information, where the size capability information indicates whether a size of the inflatable member is compatible with the patient and indicates a sizing option for the inflatable member for the patient.

The inflatable member is implanted into a patient. In some examples, the implanted inflatable member may have a particular size (e.g., one of the sizing options). In some examples, the implanted inflatable member is a test member, where the test member has a size that is large enough to encompass a plurality of sizing options. In some examples, the electronic pump assembly and the fluid reservoir are also implanted into the body of the patient. In some examples, to determine the sizing option for the patient, the electronic pump assembly and/or the fluid reservoir are not implanted into the body of the patient.

To determine the size capability information, the controller actuates at least one of a pump or an active valve to inflate the inflatable member. For example, the controller may receive a wireless signal from an external device to start an inflation cycle, which the controller sends control signals to the pump and/or the active valve to transfer fluid to the inflatable member. The controller may detect a value of at least one fluid transfer parameter for the pressure of the inflatable member to reach a target pressure during the inflation cycle. In some examples, the at least one fluid transfer parameter is a time duration to achieve the target pressure. In some examples, the at least one fluid transfer parameter is a volume of fluid transferred to the inflatable member to achieve the target pressure. In some examples, the at least one fluid transfer parameter is a pump rate required to achieve the target pressure in a predetermined amount of time. In some examples, the at least one fluid transfer parameter includes multiple parameters such as time, volume, and/or pump rate, where the controller detects a value for the time, volume, and/or pump rate, and checks the sizing information to see what sizing option(s) are mapped to the time, volume, and/or pump rate. For example, ranges of time, pump rate, or fluidic volume required to reach a target pressure may be utilized to determine the size of the inflatable member.

The controller includes sizing information that defines one or more sizing options, where each sizing option is mapped to a range of values of the fluid transfer parameter. For example, the sizing information may indicate a first range of values to reach the target pressure for a first size option, a second range of values for a second size option, a third range of values for a third size option, and so forth.

In some examples, the inflatable member having the first size option may be implanted into the patient. The controller may detect the value of the fluid transfer parameter (e.g., the time, fluid volume transferred, or pump rate) for the pressure of the inflatable member to reach the target pressure during the inflation cycle and use the sizing information to determine whether the inflatable member having the first size option is compatible with the patient. If the detected value falls outside the range associated with the first sizing option, the size capability information may indicate that the first sizing option is not compatible with the patient. Then, another inflatable member having the second sizing option may be implanted into the body of the patient to determine if the second sizing option is compatible with the patient. If the detected value falls within the range associated with the second sizing option, the size capability information may indicate that the second sizing option is compatible with the patient.

In some examples, the inflatable member may have a size large enough to encompass a plurality of different sizing options (e.g., a test inflatable member), and the test inflatable member is implanted into the patient. The controller may detect the value of the fluid transfer parameter (e.g., the time, fluid volume transferred, or pump rate) for the pressure of the inflatable member to reach the target pressure during the inflation cycle and use the sizing information to determine to select a sizing option for the inflatable member. Then, the test inflatable member may be removed, and the inflatable member having the selected sizing option may be implanted into the body of the patient. These and other features are further explained with reference to the figures.

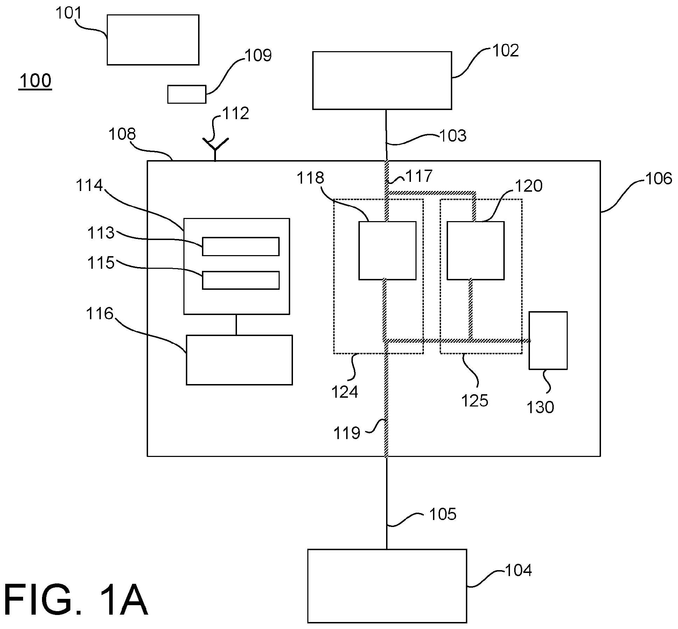

FIGS. 1A through 1C illustrate an implantable device 100 according to an aspect. In some examples, the implantable device 100 is an artificial urinary sphincter device. In some examples, the implantable device 100 is an inflatable penile prosthesis. However, the implantable device 100 may include any type of medical device that transfers fluid between components of the implantable device 100.

The implantable device 100 that can automatically determine size capability information 174 of an inflatable member 104 implanted in the body of a patient. In some examples, the size capability information 174 may indicate whether a size of the inflatable member 104 is compatible with the patient. In some examples, the size capability information 174 may indicate a sizing option 156 for the inflatable member 104 for the patient. The size capability information 174 may be transmitted to an external device 101. In some examples, the external device 101 includes a display 175 that displays the size capability information 174.

The implantable device 100 includes a fluid reservoir 102, an inflatable member 104, and an electronic pump assembly 106 configured to transfer fluid between the fluid reservoir 102 and the inflatable member 104.

The fluid reservoir 102 may include a container having an internal chamber configured to hold or house fluid that is used to inflate the inflatable member 104. In some examples, the fluid reservoir 102 is pressurized. In some examples, the fluid reservoir 102 is a pressurized balloon. In some examples, the implantable device 100 includes a single pressurized balloon. In some examples, the implantable device 100 includes two or more pressurized balloons. In some examples, the pressure differential between the inflatable member 104 and the fluid reservoir 102 will cause at least a portion of the fluid to flow from the fluid reservoir 102 to the inflatable member 104 when an active valve 118 is in the open position. In some examples, a pump 120 is activated to transfer fluid to the inflatable member 104.

The volumetric capacity of the fluid reservoir 102 may vary depending on the size of the implantable device 100. In some examples, the volumetric capacity of the fluid reservoir 102 may be 3 to 150 cubic centimeters. In some examples, the fluid reservoir 102 is constructed from the same material as the inflatable member 104. In other examples, the fluid reservoir 102 is constructed from a different material than the inflatable member 104. In some examples, the fluid reservoir 102 contains a larger volume of fluid than the inflatable member 104.

The implantable device 100 may include a first conduit connector 103 and a second conduit connector 105. Each of the first conduit connector 103 and the second conduit connector 105 may define a lumen configured to transfer the fluid to and from the electronic pump assembly 106. The first conduit connector 103 may be coupled to the electronic pump assembly 106 and the fluid reservoir 102 such that fluid can be transferred between the electronic pump assembly 106 and the fluid reservoir 102 via the first conduit connector 103. For example, the first conduit connector 103 may define a first lumen configured to transfer fluid between the electronic pump assembly 106 and the fluid reservoir 102. The first conduit connector 103 may include a single or multiple tube members for transferring the fluid between the electronic pump assembly 106 and the fluid reservoir 102.

The second conduit connector 105 may be coupled to the electronic pump assembly 106 and the inflatable member 104 such that fluid can be transferred between the electronic pump assembly 106 and the inflatable member 104 via the second conduit connector 105. For example, the second conduit connector 105 may define a second lumen configured to transfer fluid between the electronic pump assembly 106 and the inflatable member 104. The second conduit connector 105 may include a single or multiple tube members for transferring the fluid between the electronic pump assembly 106 and the inflatable member 104. In some examples, the first conduit connector 103 and the second conduit connector 105 may include a silicone rubber material. In some examples, the electronic pump assembly 106 may be directly connected to the fluid reservoir 102.

The electronic pump assembly 106 may automatically transfer fluid between the fluid reservoir 102 and the inflatable member 104 without the user manually operating a pump (e.g., squeezing and releasing a pump bulb). The electronic pump assembly 106 can control and regulate a pressure 153 within an inflatable member 104.

The electronic pump assembly 106 may include a controller 114, one or more active valves 118, one or more pumps 120, and a pressure sensor 130 (or multiple pressure sensors). The controller 114 may control the pump(s) 120 and the active valve(s) 118 to move fluid between the inflatable member 104 and the fluid reservoir 102 to transition the inflatable member 104 between an inflation state and a deflation state. The pressure sensor 130 may monitor the pressure 153 of the inflatable member 104. The controller may receive pressure readings from the pressure sensor 130 and control the pump(s) 120 and the active(s) valves 118 to maintain and/or adjust the pressure 153 of the inflatable member 104. The controller 114 may send control signals to the pump(s) 120 and the active valve(s) 118 to inflate or deflate the inflatable member 104.

In some examples, the electronic pump assembly 106 may include additional pressure sensors, which can be located at various positions in the electronic pump assembly 106, including monitoring the pressure of the fluid reservoir 102. The pressure sensor(s) 130 may be communicatively coupled to the controller 114 such that the controller 114 can receive signals (e.g., pressure readings) from the pressure sensor 130.

The electronic pump assembly 106 may include a battery 116 configured to provide power to the controller 114 and other components on the electronic pump assembly 106. In some examples, the battery 116 is a non-rechargeable battery. In some examples, the battery 116 is a rechargeable battery. In some examples, the electronic pump assembly 106 (or a portion thereof) (or the controller 114) is configured to be connected to an external charger to charge the battery 116. In some examples, the electronic pump assembly 106 may define a charging interface that is configured to connect to the external charger. In some examples, the charging interface includes a universal serial bus (USB) interface configured to receive a USB charger. In some examples, the charging technology may be electromagnetic or Piezoelectric.

The electronic pump assembly 106 may include an antenna 112 configured to wirelessly transmit (and receive) wireless signals 109 from an external device 101. The external device 101 may be any type of component that can communicate with the electronic pump assembly 106. The external device 101 may be a computer, smartphone, tablet, pendant, key fob, etc. A user may use the external device 101 to control the implantable device 100. In some examples, the user may use the external device 101 to inflate or deflate the inflatable member 104.

The external device 101 may communicate with the electronic pump assembly 106 over a network. In some examples, the network includes a short-range wireless network such as near field communication (NFC), Bluetooth, or infrared communication. In some examples, the network may include the Internet (e.g., Wi-Fi) and/or other types of data networks, such as a local area network (LAN), a wide area network (WAN), a cellular network, satellite network, or other types of data networks.

The controller 114 may be any type of controller configured to control operations of the pump 120 and/or the active valve 118. In some examples, the controller 114 is a microcontroller. In some examples, the controller 114 includes one or more drivers configured to drive the pump 120 and the active valve 118. In some examples, the driver(s) are components separate from the controller 114. The controller 114 may be communicatively coupled to the active valve 118, the pump 120, and the pressure sensor 130. In some examples, the controller 114 is connected to the active valve 118, the pump 120, and the pressure sensor 130 via wired data lines. The controller 114 may include a processor 113 and a memory device 115.

The processor 113 may be formed in a substrate configured to execute one or more machine executable instructions or pieces of software, firmware, or a combination thereof. The processor 113 can be semiconductor-based - that is, the processors can include semiconductor material that can perform digital logic. The memory device 115 may store information in a format that can be read and/or executed by the processor 113. The memory device 115 may store executable instructions that when executed by the processor 113 cause the processor 113 to perform certain operations discussed herein. The controller 114 may receive data via the pressure sensor 130 and/or the external device 101, and control the active valve 118 and/or the pump 120 by transmitting control signals to the active valve 118 and/or the pump 120.

The memory device 115 may store control parameters that can be set or modified by the user and/or physician using the external device 101. In some examples, the control parameters may include the target pressure 154 used to determine the size capability information 174. In some examples, the control parameters include a target inflation pressure and/or a partial inflation pressure. In some examples, the target inflation pressure is a maximum (or desired) pressure allowable in the inflatable member 104. In some examples, the partial inflation pressure is a pressure threshold when the inflatable member 104 is deflated. In some examples, the partial inflation pressure is zero PSI. In some examples, the partial inflation pressure is a value above zero PSI but lower than the target inflation pressure. A user or physician may update the control parameters using the external device 101, which can be communicated to the controller 114 via the antenna 112 and then updated in the memory device 115.

The inflatable member 104 may be implanted (e.g., temporarily implanted, permanently implanted) in the body of the patient. For example, if the inflatable member 104 is an inflatable cuff member, the inflatable cuff member may be implanted around the urethra. The inflatable cuff member may have an inflated state to which the inflatable cuff member restricts the urethra (e.g., to provide continence) and a deflated state in which the inflatable cuff member does not restrict the urethra (e.g., to allow for urination). If the size (e.g., diameter) of the inflatable cuff member is too small for the patient, the inflatable cuff member may restrict the urethra in the deflated stated, thereby causes the patient to strain during urination, causing residual urine in the bladder, or, in some examples, leading to urinary retention. If the size of the inflatable cuff member is too large, the inflatable cuff member may move to unintended locations on the urethra.

If the inflatable member 104 is an inflatable cylinder (or a pair of inflatable cylinders), the inflatable cylinder(s) is/are implanted into the corpora cavernosa and is/are designed to produce a rigid, erect penis when inflated. When inflated, the inflatable member 104 may extend to a state that tension is developed within a wall of the cylinder(s) to provide cylinder rigidity (and may not rely on the tunica to create the rigidity). If the size (e.g., the diameter) of the cylinder within the corporal body is too large, the desired expansion may not be obtained before contacting the tunica (which the desired rigidity of the inflatable member may not be achieved). If the size of the cylinder within the corporal body is too small, the rigidity may be developed, but close approximation of the inflatable member 104 to the implanted tissues may not be achieved (which may decrease performance results and the cosmetic appearance of the inflatable member).

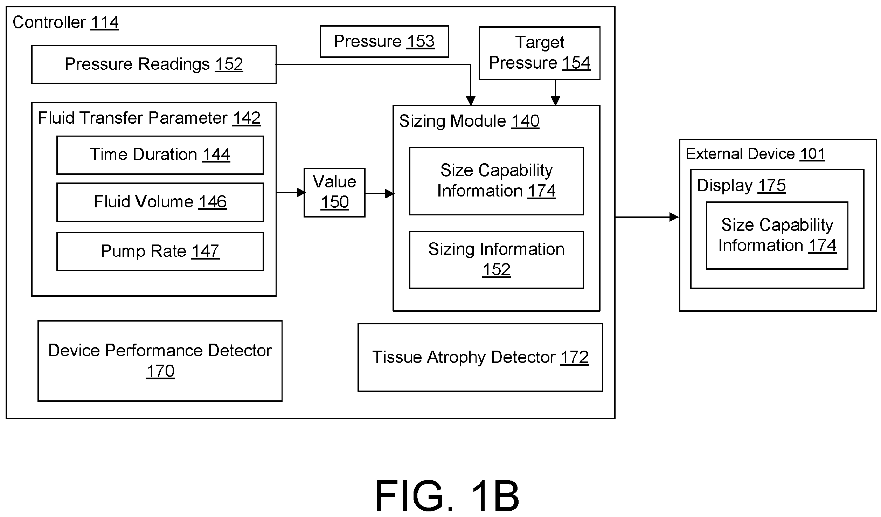

As shown in FIG. 1B, the controller 114 includes a sizing module 140 configured to generate size capability information 174, where the size capability information 174 may indicate whether a size of the inflatable member 104 is compatible with the patient and/or may indicate a sizing option 156 for the inflatable member 104 for the patient. For example, the inflatable member 104 may be implanted into a patient. In some examples, the size of the implanted inflatable member 104 is one of the sizing options 156. In some examples, the inflatable member 104 is a test member having a size that is large enough to encompass a plurality of sizing options 156.

In order to determine the size capability information 174, the inflatable member 104 is implanted into the body of the patient. In some examples, the electronic pump assembly 106 and the fluid reservoir 102 are also implanted into the body of the patient. In some examples, the fluid reservoir 102 may be implanted in the abdomen or pelvic cavity of the user (e.g., the fluid reservoir 102 may be implanted in the lower portion of the user's abdominal cavity or the upper portion of the user's pelvic cavity). In some examples, at least a portion of the electronic pump assembly 106 may be implemented in the patient's body. In some examples, to determine the size capability information 174, the electronic pump assembly 106 and/or the fluid reservoir 102 are not implanted into the body of the patient.

To determine the size capability information 174, the controller 114 may actuate at least one of a pump 120 or an active valve 118 to inflate the inflatable member 104. For example, the controller 114 may receive a wireless signal 109 from the external device 101 to start an inflation cycle, which the controller 114 sends control signals to the pump 120 and/or the active valve 118 to transfer fluid to the inflatable member 104.

The controller 114 may control the pump 120 to pump fluid between the inflatable member 104 and the fluid reservoir 102. The controller 114 may control the active valve 118 to transition between an open position (in which fluid is allowed to flow through the active valve 118) and a closed position (in which fluid is blocked from flowing through the active valve 118). In some examples, the controller 114 may control the active valve 118 to an intermediate position between the open position and the closed position, where the active valve 118 is within a partially-open state.

For example, in response to a user activating an inflation cycle using the external device 101 (e.g., selecting a user control on the external device 101), the external device 101 may transmit a wireless signal 109 to the electronic pump assembly 106 to initiate the inflation cycle (received via the antenna 112), where the controller 114 may control the pump(s) 120 and/or active valve(s) 118, thereby allowing the transfer of fluid from the fluid reservoir 102 to the inflatable member 104 until a target pressure of the inflatable member 104 is reached. In some examples, the controller 114 may control the active valve 118 to be in the open position in which the pressure differential between the fluid reservoir 102 and the inflatable member 104 causes the fluid to transfer to the inflatable member 104. In some examples, the controller 114 may control a pump 120 to operate to pump fluid into the inflatable member 104. The controller 114 may receive pressure readings from the pressure sensor 130, and, if the pressure of the inflatable member 104 has reached (or exceeded) the target pressure, the controller 114 may control the active valve 118 to be in the closed position, thereby maintaining the pressure in the inflatable member 104.

In some examples, in response to a user activating a deflation cycle using the external device 101 (e.g., selecting a user control on the external device 101), the external device 101 may transmit a wireless signal 109 to the electronic pump assembly 106 to initiate the deflation cycle (received via the antenna 112), where the controller 114 may control the active valve 118 and/or pump 120 to transfer fluid from the inflatable member 104 to the fluid reservoir 102. In some examples, the controller 114 may activate the pump 120 to transfer fluid back to the fluid reservoir 102. In some examples, the controller 114 may transition the active valve 118 to the open position to transfer fluid back to the fluid reservoir 102.

In some examples, during the deflation cycle, the active valve 118 may be in the closed position, and the controller 114 may control the pump 120 to pump the fluid from the inflatable member 104 to the fluid reservoir 102 until the pressure 153 in the inflatable member 104 reaches a lower threshold (e.g., zero pounds per square inch (PSI)). For example, the controller 114 may receive the pressure readings from the pressure sensor 130, and, in response to the detection of the pressure in the inflatable member 104 achieving the lower threshold, the controller 114 may control the pump 120 to deactivate (e.g., stop pumping).

In some examples, the electronic pump assembly 106 includes a single pump 120. The pump 120 may be disposed in parallel with the active valve 118. In some examples, the electronic pump assembly 106 includes multiple pumps 120. In some examples, one or more pumps 120 may be disposed in parallel with each other (e.g., parallel pumps). In some examples, one or more pumps 120 may be disposed in series with each other. In some examples, the electronic pump assembly 106 includes one or more parallel pumps, one or more serial pumps, or a combination of parallel pump(s) and serial pump(s).

In some examples, a pump 120 is disposed in a fluid passageway 125 that is used to empty the inflatable member 104 (e.g., during the deflation cycle). In some examples, the fluid passageway 125 is used to fill the inflatable member 104 (e.g., during the inflation cycle). In some examples, the fluid passageway 125 can be used to fill and empty the inflatable member 104. In some examples, a pump 120 is disposed in a fluid passageway 124 that is used to fill the inflatable member 104 (e.g., during the inflation cycle). In some examples, the fluid passageway 124 is used to empty the inflatable member 104. In some examples, the fluid passageway 124 is used to fill and empty the inflatable member 104. In some examples, the electronic pump assembly 108 includes a single fluid passageway having one or more pumps 120 and active valves 118. In some examples, the electronic pump assembly 108 includes more than two fluid passageways, each having a pump 120 or active valve 118 (or both). The fluid passageway 125 may include one or more pumps 120 and/or one or more active valves 118. The fluid passageway 124 may include one or more pumps 120 and/or one or more active valves 118.

The pump 120 is an electronically-controlled pump. The pump 120 may be electronically-controlled by the controller 114. For example, the pump 120 may be connected to the controller 114 and may receive a signal to actuate the pump 120. A pump 120 may be unidirectional in which the pump 120 can transfer fluid to the inflatable member 104 from the fluid reservoir 102 or to the fluid reservoir 102 from the inflatable member 104. In some examples, the pump 120 is bidirectional in which the pump 120 can transfer fluid from the fluid reservoir 102 to the inflatable member 104 and from the inflatable member 104 to the fluid reservoir 102.

In some examples, the pump 120 is an electromagnetic pump that moves the fluid from the inflatable member 104 to the fluid reservoir 102 using electromagnetism. With respect to an electromagnetic pump, a magnetic fluid is set at angles to the direction the fluid moves in, and a current is passed through it.

In some examples, the pump 120 is a piezoelectric pump. In some examples, a piezoelectric pump may be a diaphragm micropump that uses actuation of a diaphragm to drive a fluid. In some examples, a piezoelectric pump may include one or more piezo pumps (e.g., piezo elements), which may be implemented by a substrate layer (e.g., a single substrate layer) of high-voltage piezo elements or may be implemented by multiple substrate layers (e.g., stacked substrate layers) of low-voltage piezo elements. In some examples, the pump 120 includes a plurality of micro-pumps (e.g., piezoelectrically-driven micro-pumps) disposed on one or more substrates (e.g., wafer(s)). In some examples, the micro-pumps include a silicon-based material. In some examples, the micro-pumps include a metal (e.g., steel) based material. In some examples, the pump 120 is non-mechanical (e.g., without moving parts).

The active valve 118 may be an electronically-controlled valve. The active valve 118 may be electronically-controlled by the controller 114. For example, the active valve 118 may be connected to the controller 114 and may receive a signal to transition the active valve 118 between an open position in which the fluid flows through the active valve 118 and a closed position in which the fluid is prevented from flowing through the active valve 118. In some examples, the active valve 118 may be actuated to an intermediate position between the open position and the closed position in which the active valve 118 is partially open. When the active valve 118 is in the partially open position, fluid flows through the valve at a rate that is less than the fluid transfer rate of a fully open valve.

In some examples, the active valve 118 is disposed in a fluid passageway 124 that is used to fill the inflatable member 104 (e.g., in the inflation cycle). In some examples, the active valve 118 may transition to the closed position to hold (e.g., substantially hold) the pressure in the inflatable member 104. In some examples, the active valve 118 may transition to the open position to transfer fluid back to the inflatable member 104.

In some examples, the electronic pump assembly 106 includes a single active valve 118. In some examples, the electronic pump assembly 106 includes multiple active valves 118. In some examples, one or more additional active valves 118 may be in series with the pump 120. In some examples, an additional active valve 118 (e.g., a series active valve 118) may be disposed in a fluid pathway portion 117 that is connected to the fluid reservoir 102. In some examples, an additional active valve 118 (e.g., a series active valve 118) may be disposed in a fluid pathway portion 119 that is connected to the inflatable member 104. These additional active valves 118 may reduce leakage when at maximum inflation pressure.

Referring to FIG. 1B, the controller 114 may detect a value 150 of a fluid transfer parameter 142 for the pressure 153 of the inflatable member 104 to reach a target pressure 154 during the inflation cycle. In some examples, the fluid transfer parameter 142 is a time duration 144 to achieve the target pressure 154. In some examples, the fluid transfer parameter is a fluid volume 146 transferred to the inflatable member 104 to achieve the target pressure 154. In some examples, the fluid transfer parameter 142 is a pump rate 147 required to achieve the target pressure 154 in a predetermined amount of time. For example, ranges of time, pump rate, or fluidic volume required to reach the target pressure 154 may be utilized to determine the size of the inflatable member 104.

In some examples, the controller 114 may determine a first-time in which the inflation cycle is activated, receive the pressure readings from the pressure sensor 130, and determine a second time in which the pressure 153 achieves the target pressure 154. In some examples, the value 150 is the time duration from the first time to the second time. In some examples, the target pressure 154 is a threshold level in which the inflatable member 104 contacts the tissue. In some examples, the target pressure 154 is a value above zero PSI but below the maximum pressure threshold.

In some examples, the controller 114 may initiate the inflation cycle and measure the amount of fluid transferred to the inflatable member 104. In some examples, the pressure sensor 130 can also measure the amount of fluid transferred to the inflatable member using the pressure readings. After the pressure 153 achieves the target pressure 154, the controller 114 may determine the amount of fluid transferred from a first time that the inflation cycle was activated to a second time in which the pressure 153 reaches the target pressure 154. The value 150 may indicate the volume of fluid transferred from the first time to the second time.

In some examples, the controller 114 may control the rate at which the pump 120 moves fluid through the pump 120. For example, the controller 114 may operate the pump 120 according to a first pumping rate, a second pumping rate, a third pumping rate, etc. In some examples, the controller 114 may activate the pump 120 at a first time to move fluid to the inflatable member 104 according to one of the pumping rates until a second time in which the pressure 153 reaches the target pressure 154. The value 150 may include the pumping rate from the first time to the second time.

The sizing module 140 may include sizing information 152 that defines one or more sizing options 156, where each sizing option 156 is mapped to a range 158 of values of the fluid transfer parameter 142. In some examples, a range 158 may be referred to as a window or set of values. A sizing option 156 is a particular size of an inflatable member 104. In some examples, the size of the inflatable member 104 may refer to the diameter of the inflatable member 104.

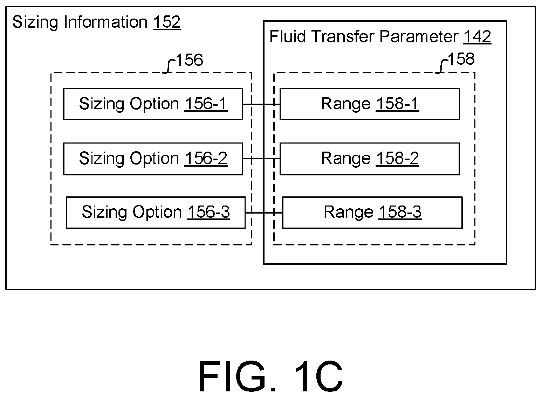

As shown in FIG. 1C, the sizing information 152 may include a sizing option 156-1 that is mapped to a range 158-1 of values of one or more fluid transfer parameters 142. In some examples, each sizing option 156 may be mapped to one fluid transfer parameter 142 or multiple fluid transfer parameters 142 (e.g., both time and volume ranges). The range 158-1 may include two or more values of the fluid transfer parameter 142 that would be compatible with an inflatable member 104 having the sizing option 156-1. The sizing information 152 may include a sizing option 156-2 that is mapped to a range 158-2 of values of the fluid transfer parameter 142. The sizing option 156-2 is a different size than the sizing option 156-1. The range 158-2 may include two or more values of the fluid transfer parameter 142 that would be compatible with an inflatable member 104 having the sizing option 156-2. The range 158-2 includes values that are different from the values of the range 158-1.

The sizing information 152 may include a sizing option 156-3 that is mapped to a range 158-3 of values of the fluid transfer parameter 142. The sizing option 156-3 is a different size than the sizing option 156-1 or the sizing option 156-2. The range 158-3 may include two or more values of the fluid transfer parameter 142 that would be compatible with an inflatable member 104 having the sizing option 156-3. The range 158-3 includes values that are different from the values of the range 158-1 or the range 158-2. Although three sizing options 156 are depicted in FIG. 1C, the sizing information 152 may define ranges 158 for any number of sizing options 156 including one sizing option 156, two sizing options 156, or more than three sizing options 156.

For example, if the fluid transfer parameter 142 is the time duration 144, the sizing information 152 may indicate a first time window (e.g., the range 158-1) to reach the target pressure 154 for the sizing option 156-1, a second time window (e.g., the range 158-2) for the sizing option 156-2, a third time window (e.g., the range 158-3) for the sizing option 156-3, and so forth. Instead of using the time duration 144, the sizing option(s) 156 may be mapped to different ranges of fluid volumes 146 to reach the target pressure 154 or mapped to different ranges of pump rates 147 to reach the target pressure 154 in a predetermined period of time.

In some examples, the inflatable member 104 having the sizing option 156-1 may be implanted into the patient. The sizing module 140 may detect the value 150 of the fluid transfer parameter 142 (e.g., the time duration 144, the fluid volume 146, or the pump rate 147) for the pressure 153 of the inflatable member 104 to reach the target pressure 154 during the inflation cycle and use the sizing information 152 to determine whether the inflatable member 104 having the sizing option 156-1 is compatible with the patient.

If the detected value 150 falls outside the range 158-1 associated with the sizing option 156-1, the size capability information 174 may indicate that the sizing option 156-1 is not compatible with the patient. Then, another inflatable member 104 having the sizing option 156-2 may be implanted into the body of the patient to determine if the sizing option 156-2 is compatible with the patient. If the detected value 150 falls within the range 158-2 associated with the sizing option 156-2, the size capability information 174 may indicate that the sizing option 156-2 is compatible with the patient.

In some examples, the inflatable member 104 may have a size large enough to encompass a plurality of different sizing options 156 (e.g., a test inflatable member), and the test inflatable member is implanted into the patient. The controller may detect the value 150 of the fluid transfer parameter 142 (e.g., the time duration 144, the fluid volume 146, or the pump rate 147) for the pressure 153 of the inflatable member 104 to reach the target pressure 154 during the inflation cycle and use the sizing information 152 to determine to select a sizing option 156 for the inflatable member 104. Then, the test inflatable member 104 may be removed, and the inflatable member 104 having the selected sizing option 156 may be implanted into the body of the patient.

In some examples, the controller 114 includes a device performance detector 170 configured to detect a performance of the implantable device 100 after the inflatable member 104 (having the appropriate size) is implanted into the body of the patient. The device performance detector 170 may detect a performance issue event based on the value 150 of the fluid transfer parameter 142 for the pressure 153 of the inflatable member 104 to reach the target pressure 154 during at least a portion of the inflation cycle. For example, while the implantable device 100 is in use, the device performance detector 170 may periodically detect the value 150 and use the sizing information 152 (or other information) to determine whether there is a performance issue with the implantable device 100. For example, if the detected value 150 falls outside the range 158 associated with the sizing option 156 of the inflatable member 104, the controller 114 may detect a performance issue event and send a notification to one or more external devices (including external device 101).

In some examples, the device performance detector 170 may detect a performance issue event based on pressure readings from the pressure sensor 130. For example, leaks (e.g., a leakage event) may be detected based on the pressure readings from the pressure sensor 130. In addition, pump and active valve performance may be inferred from the pressure readings from the pressure sensor 130. For example, the pressure readings may indicate that the pump(s) 120 and/or the active valve(s) 118 are not operating properly. In some examples, the controller 114 may detect a performance issue if a pump takes longer than a threshold amount of time to reach a certain pressure. For example, the controller 114 may use the pressure readings from the pressure sensor 130 along with a time scale or range 158 to detect a performance issue with one or more of the pumps 120. In response to the detection of a performance issue based on the pressure readings, a notification message may be transmitted over the network.

In some examples, the controller 114 includes a tissue atrophy detector 172 configured to detect a tissue atrophy event based on the value 150 of the fluid transfer parameter 142 for the pressure 153 of the inflatable member 104 to reach the target pressure 154 during at least a portion of the inflation cycle. In some examples, if the value 150 is greater than a threshold level that indicates tissue atrophy, the tissue atrophy detector 172 may send a notification message over the network to one or more external devices (including external device 101). In some examples, the controller 114 is configured to detect a tissue damage event (e.g., tunica has stretched or ruptured) based on the value 150 of the fluid transfer parameter 142 for the pressure 153 of the inflatable member 104 to reach the target pressure 154 during at least a portion of the inflation cycle. In some examples, if the value 150 is greater than a threshold level that indicates tissue damage (e.g., tissue rapture), the controller 114 may send a notification message over the network to one or more external devices (including external device 101).

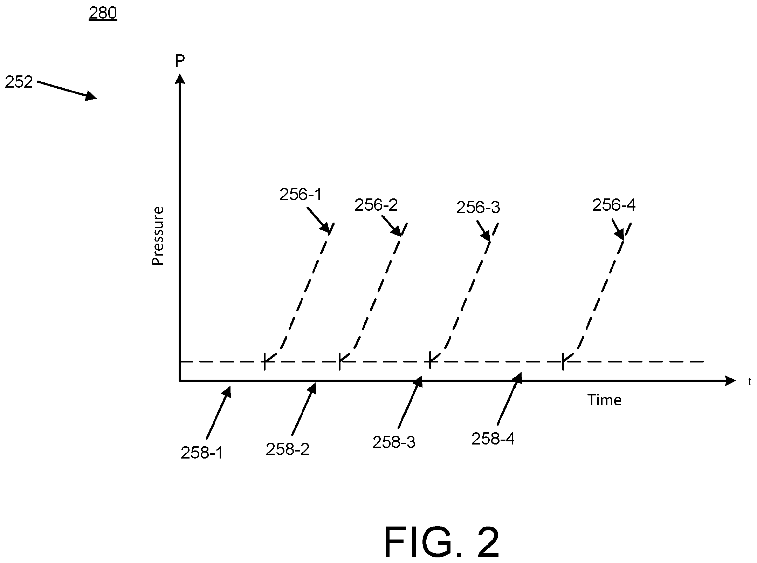

FIG. 2 illustrates a pressure profile 280 depicting sizing information 252 for a plurality of sizing options such as sizing option 256-1, sizing option 256-2, sizing option 256-3, and sizing option 256-4. The pressure profile 280 depicts increasing values for the pressure on the y-axis and increasing values for the fluid transfer parameter on the x-axis. In the pressure profile 280, the fluid transfer parameter is the time duration. However, the fluid transfer parameter may be the fluid volume or the pump rate. The sizing option 256-1 is associated with a time window 258-1. The sizing option 256-2 is associated with a time window 258-2. The sizing option 256-3 is associated with a time window 258-3. The sizing option 256-4 is associated with a time window 258-4.

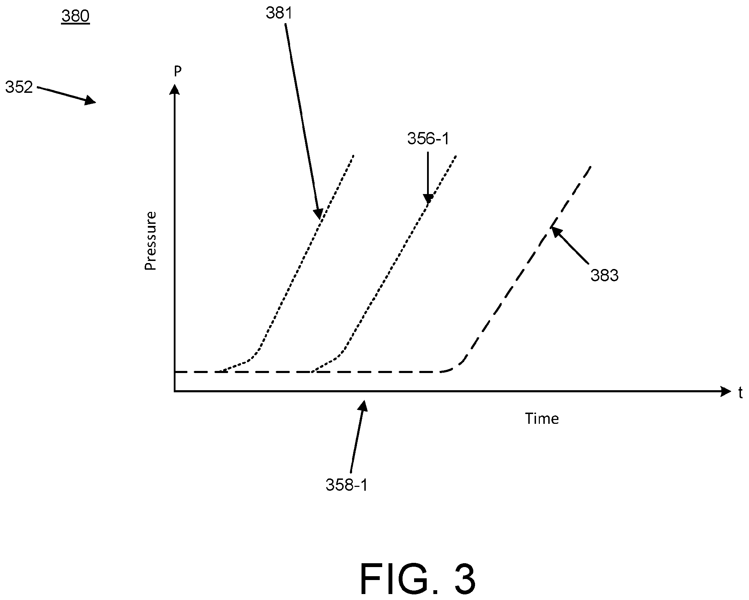

FIG. 3 illustrates a pressure profile 380 depicting sizing information 352 for a sizing option 356-1. The pressure profile 380 depicts increasing values for the pressure on the y-axis and increasing values for the fluid transfer parameter on the x-axis. In the pressure profile 380, the fluid transfer parameter is the time duration. However, the fluid transfer parameter may be the fluid volume or the pump rate. Pressure curve 381 indicates an undersized inflatable member, and pressure curve 383 indicates an oversized inflatable member. The sizing option 356-1 is associated with a time window 358-1 (e.g., between pressure curve 381 and pressure curve 383).

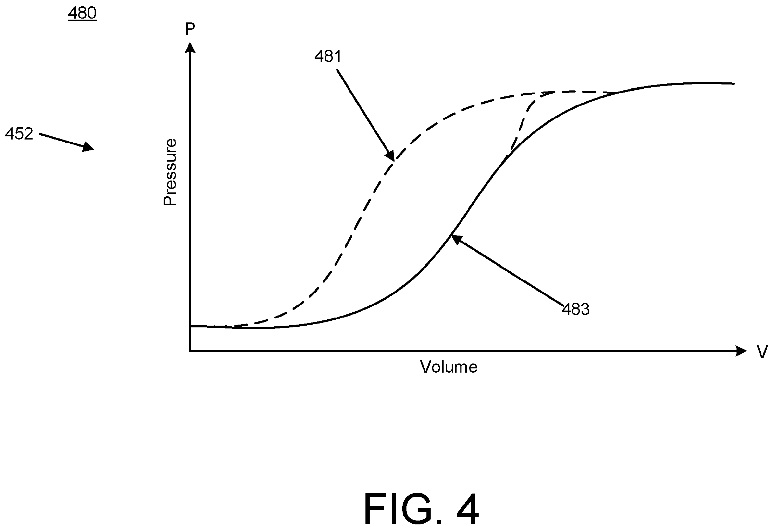

FIG. 4 illustrates a pressure profile 480 depicting sizing information 452 for a sizing option of a penile prosthesis. The pressure profile 480 depicts increasing values for the pressure on the y-axis and increasing values for the fluid transfer parameter on the x-axis. In the pressure profile 480, the fluid transfer parameter is the fluid volume. However, the fluid transfer parameter may be the time duration or the pump rate. Pressure curve 481 indicates an oversized inflatable member, and pressure curve 483 indicates an appropriately sized inflatable member.

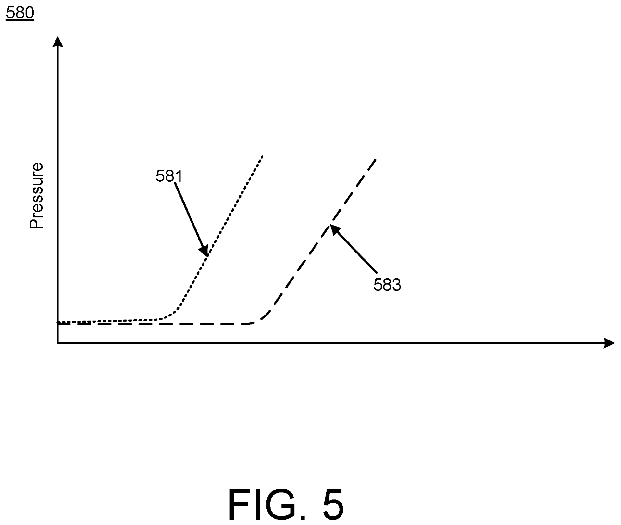

FIG. 5 illustrates a pressure profile 580 depicting a pressure curve 583 indicating a tissue atrophy event. In some examples, the tissue atrophy detector 172 of FIGS. 1A through 1C may use the pressure profile 580 to detect the tissue atrophy event. The pressure profile 580 depicts increasing values for the pressure on the y-axis and increasing values for the fluid transfer parameter on the x-axis. The fluid transfer parameter may be the time duration, the fluid volume, or the pump rate. Pressure curve 581 may indicate an appropriately working (and sized) implantable device, but pressure curve 583 may indicate a potential tissue atrophy event.

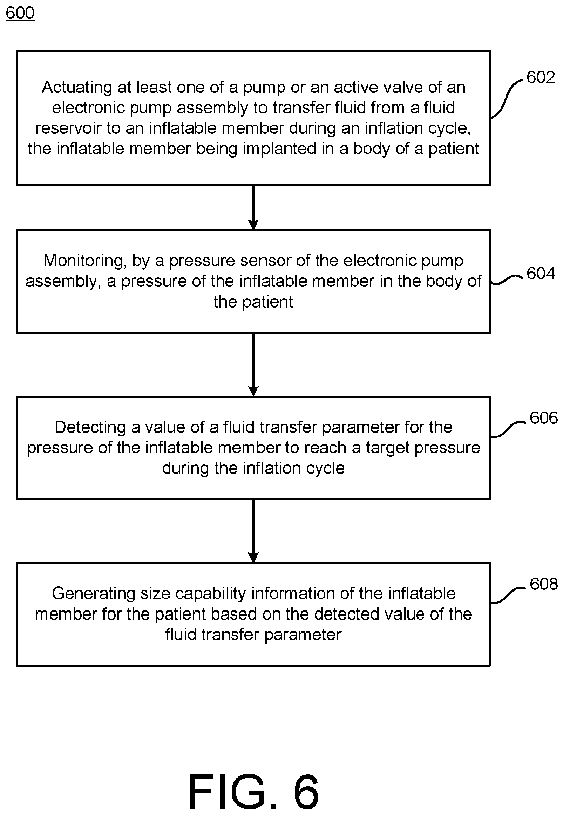

FIG. 6 illustrates a flow chart 600 depicting example operations of sizing an inflatable member of an implantable device according to an aspect. Although the flow chart 600 is explained with reference to the implantable device 100 of FIGS. 1A through 1C, the example operations of the flow chart 600 may be performed by any of the devices discussed herein.

Operation 602 includes actuating at least one of a pump 120 or an active valve 118 of an electronic pump assembly 106 to transfer fluid from a fluid reservoir 102 to an inflatable member 104 during an inflation cycle. The inflatable member 104 is implanted in the body of a patient. Operation 604 includes monitoring, by a pressure sensor 130 of the electronic pump assembly 106, a pressure 153 of the inflatable member 104 in the body of the patient. Operation 606 includes detecting a value 150 of a fluid transfer parameter 142 for the pressure 153 of the inflatable member 104 to reach a target pressure 154 during the inflation cycle. Operation 608 includes generating size capability information 174 of the inflatable member 104 for the patient based on the detected value 150 of the fluid transfer parameter 142.

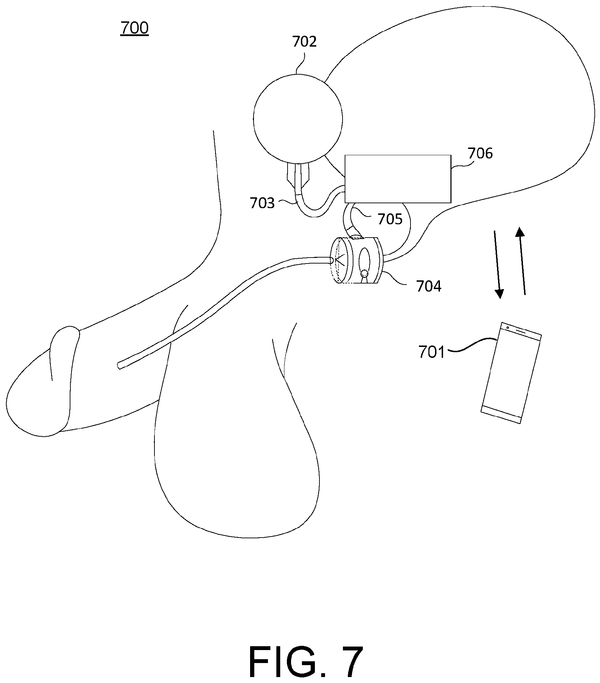

FIG. 7 illustrates a urinary control device 700 having an electronic pump assembly 706 according to an aspect. The electronic pump assembly 706 may include any of the features of the electronic pump assembly and the implantable devices discussed herein. The urinary control device 700 includes a pump assembly 706, a fluid reservoir 702, and a cuff 704.

The fluid reservoir 702 may be a pressure-regulating inflation balloon or element. The fluid reservoir 702 is in operative fluid communication with the cuff 704 via one or more tube members 703, 705. The fluid reservoir 702 is constructed of polymer material that is capable of elastic deformation to reduce fluid volume within the fluid reservoir 702 and push fluid out of the fluid reservoir 702 and into the cuff 704. However, the material of the fluid reservoir 702 can be biased or include a shape memory construct adapted to generally maintain the fluid reservoir 702 in its expanded state with a relatively constant fluid volume and pressure. In some examples, this constant level of pressure exerted from the fluid reservoir 702 to the cuff 704 will keep the cuff 704 at a desired inflated state when open fluid communication is provided between the fluid reservoir 702 and the cuff 704. This is largely due to the fact that only a small level of fluid displacement is required to inflate or deflate the cuff 704. In some examples, the fluid reservoir 702 is implanted into the abdominal space.

A user may use an external device 701 to control the urinary control device 700. In some examples, the user may use the external device 701 to inflate or deflate the cuff 704. For example, in response to the user activating an inflation cycle using the external device 701, the external device 701 may transmit a wireless signal to the electronic pump assembly 706 to initiate the inflation cycle to transfer fluid from the fluid reservoir 702 to the cuff 704 (e.g., by opening an active valve where the pressure in the fluid reservoir 702 causes the fluid to move through the active valve to the cuff 704). In some examples, in response to the user activating a deflation cycle using the external device 701, the external device 701 may transmit a wireless signal to the electronic pump assembly 706 to initiate the deflation cycle to transfer fluid from the cuff 704 to the fluid reservoir 702 using a pump.

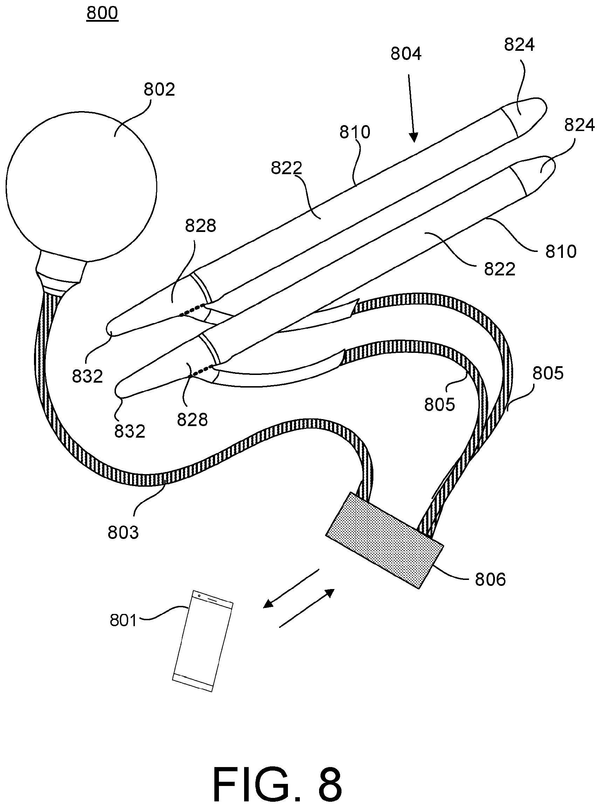

FIG. 8 schematically illustrates an inflatable penile prosthesis 800 having an electronic pump assembly 806 according to an aspect. The electronic pump assembly 806 may include any of the features of the electronic pump assembly and the implantable devices discussed herein. The inflatable penile prosthesis 800 may include an inflatable member 804 (e.g., a pair of inflatable cylinders 810), and the inflatable cylinders 810 are configured to be implanted in a penis. For example, one of the inflatable cylinders 810 may be disposed on one side of the penis, and the other inflatable cylinder 810 may be disposed on the other side of the penis. Each inflatable cylinder 810 may include a first end portion 824, a cavity or inflation chamber 822, and a second end portion 828 having a rear tip 832.

At least a portion of the electronic pump assembly 806 may be implanted in the patient's body. A pair of conduit connectors 805 may attach the electronic pump assembly 806 to the inflatable cylinders 810 such that the electronic pump assembly 806 is in fluid communication with the inflatable cylinders 810. Also, the electronic pump assembly 806 may be in fluid communication with a fluid reservoir 802 via a conduit connector 803. The fluid reservoir 802 may be implanted into the user's abdomen. The inflation chamber 822 of the inflatable cylinder 810 may be disposed within the penis. The first end portion 824 of the inflatable cylinder 810 may be at least partially disposed within the crown portion of the penis. The second end portion 828 may be implanted into the patient's pubic region PR with the rear tip 832 proximate to the pubic bone PB.

In order to implant the inflatable cylinders 810, the surgeon first prepares the patient. The surgeon often makes an incision in the penoscrotal region, e.g., where the base of the penis meets with the top of the scrotum. From the penoscrotal incision, the surgeon may dilate the patient's corpus cavernosum to prepare the patient to receive the inflatable cylinders 810. The corpus cavernosum is one of two parallel columns of erectile tissue forming the dorsal part of the body of the penis, e.g., two slender columns that extend substantially the length of the penis. The surgeon will also dilate two regions of the pubic area to prepare the patient to receive the second end portion 828. The surgeon may measure the length of the corpora cavernosum from the incision and the dilated region of the pubic area to determine an appropriate size of the inflatable cylinders 810 to implant.

After the patient is prepared, the inflatable penile prosthesis 800 is implanted into the patient. The tip of the first end portion 824 of each inflatable cylinder 810 may be attached to a suture. The other end of the suture may be attached to a needle member (e.g., Keith needle). The needle member is inserted into the incision and into the dilated corpus cavemosum. The needle member is then forced through the crown of the penis. The surgeon tugs on the suture to pull the inflatable cylinder 810 into the corpus cavernosum. This is done for each inflatable cylinder 810 of the pair. Once the inflation chamber 822 is in place, the surgeon may remove the suture from the tip. The surgeon then inserts the second end portion 828. The surgeon inserts the rear end of the inflatable cylinder 810 into the incision and forces the second end portion 828 toward the pubic bone PB until each inflatable cylinder 810 is in place.

A user may use an external device 801 to control the inflatable penile prosthesis 800. In some examples, the user may use the external device 801 to inflate or deflate the inflatable cylinders 810. For example, in response to the user activating an inflation cycle using the external device 801, the external device 801 may transmit a wireless signal to the electronic pump assembly 806 to initiate the inflation cycle to transfer fluid from the fluid reservoir 802 to the inflatable cylinders 810. In some examples, in response to the user activating a deflation cycle using the external device 801, the external device 801 may transmit a wireless signal to the electronic pump assembly 806 to initiate the deflation cycle to transfer fluid from the inflatable cylinders 810 to the fluid reservoir 802. In some examples, during the deflation cycle, fluid is transferred back until the pressure in the inflatable cylinders 810 reaches a partial inflation pressure.

Detailed embodiments are disclosed herein. However, it is understood that the disclosed embodiments are merely examples, which may be embodied in various forms. Therefore, specific structural and functional details disclosed herein are not to be interpreted as limiting, but merely as a basis for the claims and as a representative basis for teaching one skilled in the art to variously employ the embodiments in virtually any appropriately detailed structure. Further, the terms and phrases used herein are not intended to be limiting, but to provide an understandable description of the present disclosure.

## Claims

1. An implantable device (100) comprising:
a fluid reservoir (102) configured to hold fluid;
an inflatable member (104) configured to be implanted in a body of a patient; and
an electronic pump assembly (106) including:
a controller (114) configured to actuate at least one of a pump (120) or an active valve (118) to inflate the inflatable member (104); and
a pressure sensor (130) configured to monitor a pressure of the inflatable member (104),
the controller (114) configured to detect a value of at least one fluid transfer parameter (142) for the pressure of the inflatable member (104) to reach a target pressure during an inflation cycle, **characterised by** a controller (114) configured to generate size capability information (174) of the inflatable member (104) for the patient based on the detected value of the at least one fluid transfer parameter (142),
wherein the size capability information (174) indicates whether a size of the inflatable member (104) is compatible with the patient,
wherein the controller (114) includes a memory device (115) storing sizing information (152), the sizing information (152) defining at least one sizing option (156), the at least one sizing option (156) being mapped to a range of values (158) of the at least one fluid transfer parameter (142),
wherein the controller (114) is configured to determine the size capability information (174) based on whether the detected value is included within the range of values (158) of the at least one fluid transfer parameter (142).

2. The implantable device (100) of claim 1, wherein the controller (114) is configured to transmit the size capability information (174) to an external device (101).

3. The implantable device (100) of any of claims 1 to 2 , wherein the at least one fluid transfer parameter (142) includes a time duration to achieve the target pressure.

4. The implantable device (100) of any of claims 1 to 2 , wherein the at least one fluid transfer parameter (142) includes a volume of the fluid transferred to the inflatable member (104) to achieve the target pressure.

5. The implantable device (100) of any of claims 1 to 2 , wherein the at least one fluid transfer parameter (142) includes a pump rate required to achieve the target pressure in a predetermined amount of time.

6. The implantable device (100) of any of claims 1 to 5 wherein the controller (114) is configured to detect a performance issue event based on the value of the at least one fluid transfer parameter (142) for the pressure of the inflatable member (104) to reach the target pressure during the inflation cycle.

7. The implantable device (100) of any of claims 1 to 6 , wherein the implantable device (100) includes a urinary sphincter device.

8. The implantable device (100) of any of claims 1 to 7 , wherein the implantable device includes an inflatable penile prosthesis.

9. The implantable device (100) of any of the preceding claims, wherein the at least one sizing option (156) includes a first sizing option (256-1) and a second sizing option (256-2), the first sizing option (256-1) being mapped to a first range of values (258-1), the second sizing option (256-2) being mapped to a second range of values (258-2), the controller (114) configured to select the first sizing option (256-1) in response to the detected value belonging to the first range of values (258-1).

10. The implantable device of any of the preceding claims, wherein the controller (114) is configured to detect a tissue atrophy event or tissue damage based on the value of the at least one fluid transfer parameter (142) for the pressure of the inflatable member (104) to reach the target pressure during the inflation cycle.

11. The implantable device of any of the preceding claims, wherein the controller (114) is configured to transmit the selected sizing option to an external device (101) for display.

## Patentansprüche

1. Implantierbare Vorrichtung (100), umfassend:
einen Fluidbehälter (102), dazu eingerichtet, Fluid zu halten;
ein fluidexpandierbares Element (104), das eingerichtet ist, in einen Körper eines Patienten implantiert werden zu können; und
eine elektronische Pumpenanordnung (106), die umfasst:
eine Steuerung (114), die eingerichtet ist, mindestens eine Pumpe (120) oder ein aktives Ventil (118) zu betätigen, um das fluidexpandierbare Element (104) zu expandieren; und
einen Drucksensor (130), der eingerichtet ist, einen Druck des fluidexpandierbaren Elements (104) zu überwachen,
die Steuerung (114) eingerichtet ist, einen Wert mindestens eines Fluidtransferparameters (142) zu erfassen, damit der Druck des fluidexpandierbaren Elements (104) einen Zieldruck während eines Expansionszyklus erreicht, **gekennzeichnet durch** eine Steuerung (114), die eingerichtet ist, Größenfähigkeitsinformationen (174) des fluidexpandierbaren Elements (104) für den Patienten auf der Grundlage des erfassten Werts des mindestens einen Fluidtransferparameters (142) zu erzeugen,
wobei die Größenfähigkeitsinformation (174) anzeigt, ob eine Größe des fluidexpandierbaren Elements (104) mit dem Patienten kompatibel ist,
wobei die Steuerung (114) eine Speichervorrichtung (115) enthält, die Größeninformationen (152) speichert, wobei die Größeninformationen (152) mindestens eine Größenoption (156) definieren, wobei die mindestens eine Größenoption (156) auf einen Wertebereich (158) des mindestens einen Fluidtransferparameters (142) abgebildet wird,
wobei die Steuerung (114) eingerichtet ist, die Größenfähigkeitsinformationen (174) auf der Grundlage zu bestimmen, ob der erfasste Wert innerhalb des Wertebereichs (158) des mindestens einen Fluidtransferparameters (142) enthalten ist.

2. Implantierbare Vorrichtung (100) nach Anspruch 1, wobei die Steuerung (114) eingerichtet ist, die Größenfähigkeitsinformationen (174) an eine externe Vorrichtung (101) zu übermitteln.

3. Implantierbare Vorrichtung (100) nach einem der Ansprüche 1 bis 2, wobei der mindestens eine Fluidtransferparameter (142) eine Zeitdauer zum Erreichen des Zieldrucks umfasst.

4. Implantierbare Vorrichtung (100) nach einem der Ansprüche 1 bis 2, wobei der mindestens eine Fluidübertragungsparameter (142) ein Volumen des Fluids einschließt, das zu dem fluidexpandierbaren Element (104) übertragen wird, um den Zieldruck zu erreichen.

5. Implantierbare Vorrichtung (100) nach einem der Ansprüche 1 bis 2, wobei der mindestens eine Fluidtransferparameter (142) eine Pumprate umfasst, die erforderlich ist, um den Zieldruck in einer vorgegebenen Zeitspanne zu erreichen.

6. Implantierbare Vorrichtung (100) nach einem der Ansprüche 1 bis 5, wobei die Steuerung (114) eingerichtet ist, ein Leistungsproblemereignis basierend auf dem Wert des mindestens einen Fluidtransferparameters (142) zu erkennen, damit der Druck des fluidexpandierbaren Elements (104) den Zieldruck während des Expansionszyklus erreicht.

7. Implantierbare Vorrichtung (100) nach einem der Ansprüche 1 bis 6, wobei die implantierbare Vorrichtung (100) eine Harnschließmuskelvorrichtung enthält.

8. Implantierbare Vorrichtung (100) nach einem der Ansprüche 1 bis 7, wobei die implantierbare Vorrichtung eine fluidexpandierbare Penisprothese enthält.

9. Implantierbare Vorrichtung (100) nach einem der vorhergehenden Ansprüche, wobei die mindestens eine Größenoption (156) eine erste Größenoption (256-1) und eine zweite Größenoption (256-2) umfasst, wobei die erste Größenoption (256-1) auf einen ersten Wertebereich (258-1) abgebildet ist, die zweite Größenoption (256-2) einem zweiten Wertebereich (258-2) abgebildet ist, wobei die Steuerung (114) eingerichtet ist, die erste Größenoption (256-1) in Reaktion auf den erfassten Wert, der zu dem ersten Wertebereich (258-1) gehört, auszuwählen.

10. Implantierbare Vorrichtung nach einem der vorhergehenden Ansprüche, wobei die Steuerung (114) eingerichtet ist, ein Gewebeatrophieereignis oder eine Gewebeschädigung basierend auf dem Wert des mindestens einen Fluidtransferparameters (142) zu erkennen, damit der Druck des fluidexpandierbaren Elements (104) den Zieldruck während des Expansionszyklus erreicht.

11. Implantierbare Vorrichtung nach einem der vorhergehenden Ansprüche, wobei die Steuerung (114) eingerichtet ist, die ausgewählte Größenoption zur Anzeige an eine externe Vorrichtung (101) zu übermitteln.

## Revendications

1. Dispositif implantable (100) comprenant :
un réservoir de fluide (102) configuré pour contenir du fluide ;
un élément gonflable (104) configuré pour être implanté dans le corps d'un patient ; et
un assemblage de pompe électronique (106) incluant :
un contrôleur (114) configuré pour actionner au moins un composant parmi une pompe (120) et une soupape active (118) afin de gonfler l'élément gonflable (104) ; et
un capteur de pression (130) configuré pour surveiller une pression de l'élément gonflable (104),
le contrôleur (114) étant configuré pour détecter une valeur d'au moins un paramètre de transfert de fluide (142) pour la pression de l'élément gonflable (104) afin qu'il atteigne une pression cible pendant un cycle de gonflage, **caractérisé en ce que** un contrôleur (114) est configuré pour générer une information de capacité dimensionnelle (174) de l'élément gonflable (104) pour le patient sur la base de la valeur détectée de l'au moins un paramètre de transfert de fluide (142),
dans lequel l'information de capacité dimensionnelle (174) indique si une dimension de l'élément gonflable (104) est ou non compatible avec le patient,
dans lequel le contrôleur (114) inclut un dispositif de mémoire (115) qui stocke une information de dimensionnement (152), l'information de dimensionnement (152) définissant au moins une option de dimensionnement (156), l'au moins une option de dimensionnement (156) étant cartographiée par rapport à une plage de valeurs (158) de l'au moins un paramètre de transfert de fluide (142), et
dans lequel le contrôleur (114) est configuré pour déterminer l'information de capacité dimensionnelle (174) sur la base de si la valeur détectée est incluse à l'intérieur de la plage de valeurs (158) de l'au moins un paramètre de transfert de fluide (142).

2. Dispositif implantable (100) selon la revendication 1, dans lequel le contrôleur (114) est configuré pour transmettre l'information de capacité dimensionnelle (174) à un dispositif externe (101).

3. Dispositif implantable (100) selon l'une quelconque des revendications 1 et 2, dans lequel l'au moins un paramètre de transfert de fluide (142) inclut une durée temporelle pour atteindre la pression cible.

4. Dispositif implantable (100) selon l'une quelconque des revendications 1 et 2, dans lequel l'au moins un paramètre de transfert de fluide (142) inclut un volume du fluide qui est transféré sur l'élément gonflable (104) pour atteindre la pression cible.

5. Dispositif implantable (100) selon l'une quelconque des revendications 1 et 2, dans lequel l'au moins un paramètre de transfert de fluide (142) inclut un débit de pompe qui est requis pour atteindre la pression cible dans une quantité de temps prédéterminée.

6. Dispositif implantable (100) selon l'une quelconque des revendications 1 à 5, dans lequel le contrôleur (114) est configuré pour détecter un événement de problème de performance sur la base de la valeur de l'au moins un paramètre de transfert de fluide (142) pour que la pression de l'élément gonflable (104) atteigne la pression cible pendant le cycle de gonflage.

7. Dispositif implantable (100) selon l'une quelconque des revendications 1 à 6, dans lequel le dispositif implantable (100) inclut un dispositif de sphincter urinaire.

8. Dispositif implantable (100) selon l'une quelconque des revendications 1 à 7, dans lequel le dispositif implantable inclut une prothèse pénienne gonflable.

9. Dispositif implantable (100) selon l'une quelconque des revendications précédentes, dans lequel l'au moins une option de dimensionnement (156) inclut une première option de dimensionnement (256-1) et une seconde option de dimensionnement (256-2), la première option de dimensionnement (256-1) étant cartographiée par rapport à une première plage de valeurs (258-1), la seconde option de dimensionnement (256-2) étant cartographiée par rapport à une seconde plage de valeurs (258-2), le contrôleur (114) étant configuré pour sélectionner la première option de dimensionnement (256-1) en réponse au fait que la valeur détectée appartient à la première plage de valeurs (258-1).

10. Dispositif implantable selon l'une quelconque des revendications précédentes, dans lequel le contrôleur (114) est configuré pour détecter un événement d'atrophie tissulaire ou une lésion tissulaire sur la base de la valeur de l'au moins un paramètre de transfert de fluide (142) pour que la pression de l'élément gonflable (104) atteigne la pression cible pendant le cycle de gonflage.

11. Dispositif implantable selon l'une quelconque des revendications précédentes, dans lequel le contrôleur (114) est configuré pour transmettre l'option de dimensionnement sélectionnée à un dispositif externe (101) pour son affichage.
